# EUROPÄISCHE PATENTSCHRIFT

(11) **EP 2 419 257 B1**
(45) Veröffentlichungstag und Bekanntmachung des Hinweises auf die Patenterteilung: **06.03.2013**
(21) Anmeldenummer: 10713300.1
(22) Anmeldetag: 12.04.2010
(51) Int. Cl.: B29C 65/20, B29C 65/78, A61M 39/08

(54) **VORRICHTUNG ZUM VERSCHWEISSEN VON THERMOPLASTISCHEN SCHLÄUCHEN**
APPARATUS FOR WELDING TOGETHER THERMOPLASTIC HOSES
DISPOSITIF DE SOUDAGE DE TUBES THERMOPLASTIQUES

(30) Priorität: 14.04.2009 CH 5892009
(43) Veröffentlichungstag der Anmeldung: 22.02.2012
(73) Patentinhaber: Reed Electronics AG, 6105 Schachen (CH)
(72) Erfinder: BÜHLER, Peter, CH-6103 Schwarzenberg (CH); FLUDER, Willy, CH-6362 Stansstad (CH)
(74) Vertreter: Rentsch Partner AG
(86) Internationale Anmeldenummer: PCT/CH2010/000100
(87) Internationale Veröffentlichungsnummer: WO 2010/118546

(56) Entgegenhaltungen:
- EP-A2- 0 953 431
- WO-A1-2005/000565
- US-A1- 2002 174 956

## Beschreibung

### TECHNISCHES GEBIET

Die Erfindung betrifft eine Vorrichtung zum Verschweissen von thermoplastischen Schläuchen mit zwei gegeneinander in Teilen verdrehbaren und verschiebbaren, jeweils zwei Spannbacken aufweisenden Spanneinheiten mit Durchgangsöffnungen zwischen den Spannbacken, in welche die Schläuche parallel zueinander durchlaufend eingelegt und quer zueinander gequetscht werden können, und mit einer beheizbaren Klinge zum Durchschneiden der Schläuche und Aufschmelzen ihrer Schnittkanten zwischen den Spanneinheiten.

Derartige Vorrichtungen kommen zum Beispiel in der Biotechnologie und in der Medizin zur Anwendung wenn es notwendig ist, sterile Schlauch-Verbindungen z.B. zwischen einer Zellkultur und einem Nährmedium bzw. zwischen einem Dialyse-Patienten und einem Beutel für die Spüllösung herzustellen. Durch das Quetschen der Schläuche wird verhindert, dass beim Schweissprozess die in ihnen enthaltene Flüssigkeit austreten und die Umgebung ggf. kontaminieren kann. Am Ende des Schweissprozesses sind sämtliche Schlauchenden verschlossen. Man spricht hierbei auch von "Total Containment" unter Nassschweiss-Bedingungen.

### STAND DER TECHNIK

Aus EP 0 778 123 ist eine Vorrichtung der vorgenannten Art bekannt, bei welcher nach dem Durchschneiden der Schläuche eine der Spanneinheiten um 180° gedreht wird und dann die durch dieses Drehen neu in Flucht miteinander gekommenen Schlauchabschnitte miteinander verschweisst werden. In den Durchgangsöffnungen der beiden Spanneinheiten sind die Schläuche bei dieser Vorrichtung jeweils direkt übereinander gestapelt und werden darin direkt quer aufeinander gequetscht. Es ist nicht ersichtlich, wie ein Schlauch, welcher nach der Drehung und dem Verschweissen beidseitig mit einem Behälter verbunden ist, wieder aus den Spanneinheiten herausgenommen werden kann. Durch die Art der Ausbildung der Drehlagerung ist die bekannte Vorrichtung recht voluminös.

Bei der Vorrichtung von EP 0 507 321 sind ebenfalls zwei gegeneinander verdrehbare Spanneinheiten vorgesehen. In diese werden die Schläuche jeweils in zwei voneinander beabstandete Durchgangsöffnungen eingelegt und darin senkrecht zu ihrer gemeinsamen Lageebene gequetscht. Die Art der Lagerung der Spanneinheiten wird nicht beschrieben.

Eine weitere Vorrichtung ist in der EP0953431 beschrieben.

### DARSTELLUNG DER ERFINDUNG

Aufgabe der Erfindung ist es insbesondere, eine Vorrichtung der eingangs genannten Art anzugeben, welche die exakte Verschweissung von zwei flüssigkeitsgefüllten Schläuchen ohne Austritt von Flüssigkeit in die Umgebung erlaubt und welche kompakt aufgebaut ist.

Die Aufgabe wird gelöst durch eine Vorrichtung zum Verschweissen von thermoplastischen Schläuchen gemäss Anspruch 1, welche dadurch gekennzeichnet ist, dass zwischen den Spannbacken der Spanneinheiten, eingespannte Schläuche voneinander trennend, jeweils ein Zwischenstück angeordnet ist und dass mindestens eine der beiden Spanneinheiten innere sowie äussere Spannbacken aufweist, wobei die inneren Spannbacken von den äusseren Spannbacken gehalten, gemeinsam mit diesen verstellbar und in einer Schliessstellung miteinander sowie zusammen mit dem von ihnen eingeschlossenen Zwischenstück in den äusseren Spannbacken drehbar sind.

Durch das Quetschen nimmt der Durchmesser der Schläuche in Quetschrichtung ab und wird gleichzeitig quer zur Quetschrichtung grösser. Indem die Schläuche in der erfindungsgemässen Vorrichtung wie bei EP 0 778 123 quer zueinander gequetscht werden, benötigen ihre zusammengequetschten und miteinander zu verschweissenden Querschnitte weniger Platz als bei EP 0 507 321, bei der die zusammengequetschten Querschnitte längs zueinander angeordnet sind. Dies ermöglicht einen kompakteren Aufbau der Vorrichtung als bei EP 0 507 321.

Durch die erfindungsgemäss vorgesehenen Zwischenstücke sind die Schläuche im zusammengequetschten Zustand andererseits wie bei EP 0 507 321 in separaten Durchgangsöffnungen voneinander getrennt, was eine bessere und genauere Festlegung ihrer miteinander zu verschweissenden Querschnitte erlaubt und auch sicherstellt, dass es nicht zu einem unerwünschten Verschweissen benachbarter Schläuche kommt.

Zum kompakten Aufbau trägt auch wesentlich die erfindungsgemäss vorgesehene Aufteilung der Spannbacken einer Spanneinheit in innere und äussere Spannbacken bei, wobei lediglich die inneren Spannbacken in den äusseren Spannbacken drehbar ausgebildet zu sein brauchen. Es ist dadurch nicht notwendig, die gesamte Spanneinheit zu rotieren.

Die Schläuche müssen nicht über die gesamte Länge der Durchgangsöffnungen, die typischerweise zwischen 30 mm und 70 mm betragen kann, völlig zusammengequetscht werden. Es genügt, wenn dies nur in relativ kurzen, in Längsrichtung z.B. nur 1 mm und 10 Millimeter ausgedehnten Quetschzonen der Fall ist, wobei diese Quetschzonen angrenzend an die sich gegenüberliegenden Seiten der beiden Spannvorrichtungen mit geringstem gegenseitigen Abstand angeordnet sein sollten. Die restliche Länge der Durchgangsöffnungen, in der diese einen breiteren Querschnitt gegenüber den Quetschzonen aufweisen, dient nur zur ergänzenden Halterung und Führung der Schläuche und wird nachstehend als Haltezone bezeichnet.

In den Quetschzonen sind die Durchgangsöffnungen vorzugsweise mit einem rechteckigen Querschnitt versehen mit den längeren Rechteckseiten jeweils parallel zueinander ausgerichtet, wobei die jeweils inneren Rechteckseiten durch ebene Anlageflächen an Stegen der Zwischenstücke und die äusseren Rechteckseiten durch ebene Anlageflächen an stegartigen Wandungen der Spannbacken bzw. der inneren Spannbacken definiert werden. Die Breite des rechteckigen Querschnitts sollte knapp der doppelten Wandstärke der miteinander zu verschweissenden Schläuche entsprechen. Um eine bessere Verdrängung der Flüssigkeit aus den Bereichen der Quetschzone zu erreichen, kann die Breite des rechteckigen Querschnitts 50% bis 70% der doppelten Wandstärke der miteinander zu verschweissenden Schläuche entsprechen.

Die Länge der Rechteckseiten kann demgegenüber grösser als für die miteinander zu verschweissenden Schläuche jeweils erforderlich und insbesondere ausreichend lang für die dicksten der miteinander zu verbindenden Schläuche bemessen sein, wenn die Schläuche, wie durch das noch beschriebene Profil der Zwischenstücke, auf Höhe der Drehachse der inneren Spannbacken gehalten werden. Durch den rechteckigen Querschnitt der Durchgangsöffnungen ist der Schlauchquerschnitt in der Schnittebene zwischen den Spanneinheiten sehr genau festgelegt und zudem rotationssymmetrisch, was ein genaues Zusammenpassen der Schlauchquerschnitte nach dem Drehen sicherstellt.

Die bereits erwähnten Stege der Zwischenstücke können zusätzlich als Schlauchbegrenzung an beiden Anlageflächen oben und unten Vorsprünge aufweisen, so dass sich ein doppel-T-förmiges Profil der Stege ergibt. Dadurch wird die rechteckige Durchgangsöffnung im Bereich der Quetschzone in ihrer Länge begrenzt, wobei der Schlauchquerschnitt der gequetschten Schläuche in der Schnittebene zwischen den Spanneinheiten noch genauer festgelegt werden kann. Dies stellt ein noch genaueres Zusammenpassen der Schlauchquerschnitte nach dem Drehen sicher. Die Länge der rechteckigen Durchgangsöffnung im Bereich der Quetschzone beträgt vorzugsweise 120 - 140 % des Durchmessers der zu quetschenden Schläuche.

Im Bereich der Haltezonen weisen die Durchgangsöffnungen wie bereits erwähnt einen gegenüber dem Querschnitt in den Quetschzonen breiteren Querschnitt auf, wobei dieser Querschnitt vorzugsweise jeweils in Richtung weg von den Zwischenstücken verbreitert ist. Durch die Verbreiterung nach aussen kommen die rechteckigen Querschnitte in den Quetschzonen vorteilhaft eng beieinander zu liegen, was sich günstig auf den kompakten Aufbau der inneren Spannbacken auswirkt.

Durch die Verbreiterung nach aussen können die Zwischenstücke zudem im Bereich der Haltezonen mit einem doppel-c-förmigen Profil versehen sein, in welchem die Schläuche beim Einlegen in die Durchgangsöffnungen gerade durchlaufend vorfixiert werden können. Vorzugsweise ist dieses Profil an den Durchmesser der jeweils zu verschweissenden Schläuche angepasst. Das Profil hält zudem, wie bereits erwähnt, die Schläuche auf Höhe der Drehachse der inneren Spannbacken, wodurch die Länge der Rechteckseiten der Durchgangsöffnungen in den Quetschzonen grösser als für die miteinander zu verschweissenden Schläuche jeweils erforderlich bemessen sein können. Wenn zusätzlich die Spannbacken bzw. die inneren Spannbacken im Bereich der Haltezonen jeweils eine flache Anlagefläche gegenüber den Schläuchen aufweisen, genügt zur Anpassung der Vorrichtung an Schläuche mit unterschiedlichem Durchmesser das Auswechseln der Zwischenstücke.

Durch Auswechseln lediglich der Zwischenstücke lässt sich die erfindungsgemässe Vorrichtung auch an Schläuche mit unterschiedlicher Wandstärke anpassen, wobei einfach die den gegenseitigen Abstand bestimmende Dicke der Zwischenstücke bzw. ihres erwähnten Steges geeignet gewählt werden muss. Dabei können auch die Vorsprünge der Stege entsprechend dimensioniert werden, um die rechteckige Durchgangsöffnung im Bereich der Quetschzone den verschiedenen Schlauchdurchmessern anzupassen.

Die inneren Spannbacken sind vorzugsweise in den äusseren Spannbacken ausserhalb der Schliessstellung gegen Verdrehen fixiert, was das Einlegen und Herausnehmen der Schläuche sowie zumindest auch ein motorisches Schliessen der Spanneinheiten erleichtert.

Um die Bedienungsfreundlichkeit der erfindungsgemässen Vorrichtung weiter zu erhöhen, können die Zwischenstücke jeweils auf einer Halterung gehalten sein. Damit das Zwischenstück zwischen den inneren Spannbacken zusammen mit diesen drehbar ist, muss die Verbindung dieses Zwischenstücks zu seiner Halterung in der Schliessstellung gelöst sein.

Ein einfacher Aufbau der erfindungsgemässen Vorrichtung ergibt sich zudem, wenn jeweils einer der Spannbacken bzw. der äusseren Spannbacken der Spanneinheiten ortsfest montiert und zum Einlegen in bzw. Entnehmen der Schläuche aus den Durchgangsöffnungen jeweils nur der andere Spannbacken sowie die Zwischenstücke ggf. zusammen mit ihren Halterungen bewegbar montiert sind. Die Bewegung erfolgt hierbei weiter vorzugsweise motorisch entlang einer linearen Führung.

Die Spanneinheit mit den inneren und den äusseren Spannbacken ist schliesslich bevorzugt einschliesslich des in ihr angeordneten Zwischenstücks rotationssymmetrisch bezüglich halber Umdrehungen der inneren Spannbacken und des Zwischenstücks in den äusseren Spannbacken ausgebildet. Die erfindungsgemässe Vorrichtung ist dadurch nach einem Schweissvorgang und der Entnahme der miteinander verschweissten Schläuche für einen weiteren Schweissvorgang sofort wieder einsetzbar, ohne dass die inneren Spannbacken und das zwischen diesen angeordnete Zwischenstück in den äusseren Spannbacken erst wieder in seine Ausgangsstellung gedreht werden müsste.

Um bei flüssigkeitsgefüllten Schläuchen sicherzustellen, dass in den Schlauchabschnitten zwischen den beiden Spanneinheiten nach dem Quetschen der Schläuche keine Restflüssigkeit mehr enthalten ist, die beim Durchtrennen der Schläuche in die Umgebung austreten und/oder an der heissen Klinge verdampfen könnte, darf der Abstand der beiden Spanneinheiten bzw. der erwähnten Quetschzonen nicht zu gross sein. Ein geeigneter Abstand beim Durchtrennen der Schläuche ist z.B. 3 - 6 mm, vorzugsweise 5 mm. Damit sich zwischen den beiden Spanneinheiten keine flüssigkeitsgefüllten Schlauchtaschen ausbilden können, sollte das Quetschen der Schläuche an beiden Spanneinheiten zudem nicht gleichzeitig, sondern nacheinander erfolgen. Zum Verschweissen der Schläuche muss der genannte Abstand ggf. zusätzlich z.B. um einige Millimeter verringert werden können.

### KURZE ERLÄUTERUNG DER FIGUREN

Die Erfindung soll nachfolgend anhand von Ausführungsbeispielen im Zusammenhang mit der Zeichnung näher erläutert werden. Es zeigen:
- Fig. 1: eine erfindungsgemässe Vorrichtung mit zwei Spanneinheiten in perspektivischer Ansicht seiner Vorderseite;
- Fig. 2: die Vorrichtung von Fig. 1 mit eingelegten Schläuchen;
- Fig. 3: die Vorrichtung von Fig. 1 in perspektivischer Ansicht seiner Rückseite;
- Fig. 4a: eine Ansicht der Rückseite lediglich der Spannbacken und des Zwischenstücks mit eingelegten Schläuchen der vorderseitigen Spanneinheit im geöffneten Zustand;
- Fig. 4b: die Spanneinheit von Fig. 4a im geschlossenen Zustand;
- Fig. 4c: die Spanneinheit von Fig. 4b mit gedrehten inneren Spannbacken, Zwischenstück und Schläuchen;
- Fig. 5: Fig. 4a - 4c entsprechende Ansichten der Vorderseite der Spannbacken und des Zwischenstücks mit eingelegten Schläuchen der vorderseitigen Spanneinheit;
- Fig. 6a: schematisch zwei Behälter mit endseitig verschlossenen Schläuchen vor;
- Fig. 6b: nach der Verbindung der Schläuche unter Verwendung der erfindungsgemässen Vorrichtung;
- Fig. 7: eine perspektivische Ansicht eines Zwischenstücks mit doppel-T-förmigem Steg;
- Fig. 8a: eine Ansicht der Rückseite der Spannbacken und des Zwischenstücks aus Fig. 7 mit eingelegten Schläuchen der vorderseitigen Spanneinheit im geöffneten Zustand; und
- Fig. 8b: die Spanneinheit von Fig. 8a im geschlossenen Zustand.

### WEGE ZUR AUSFÜHRUNG DER ERFINDUNG

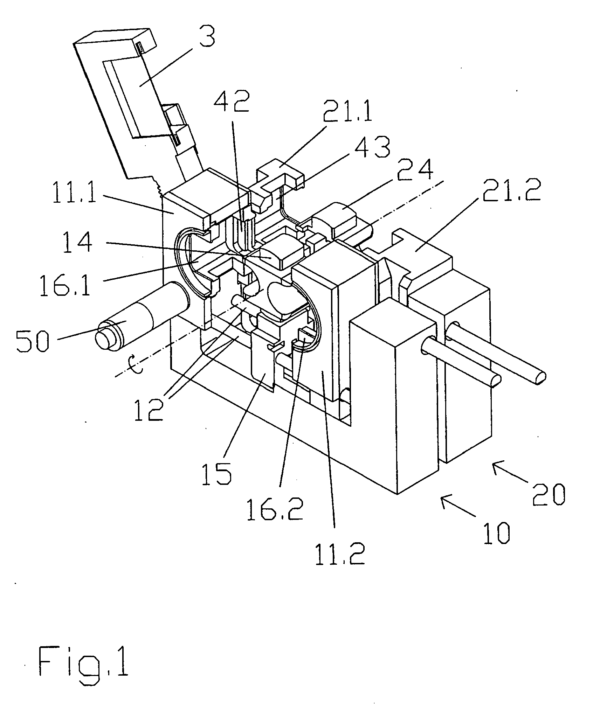

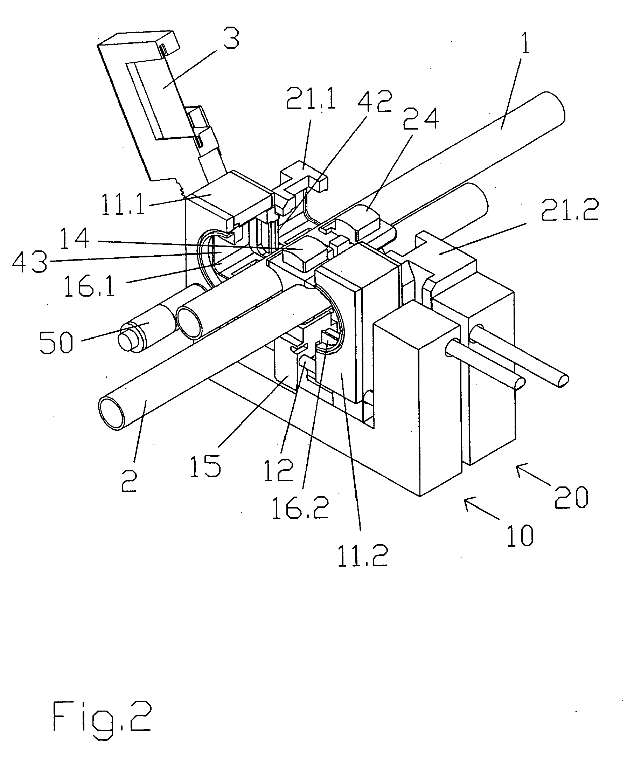

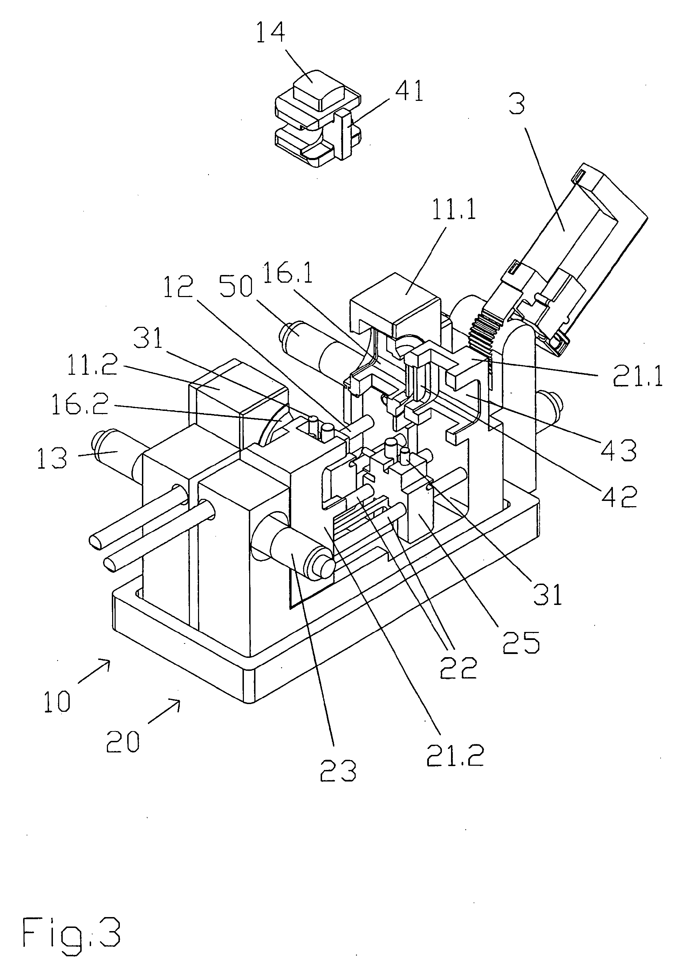

Die Figuren 1-3 zeigen eine erfindungsgemässe Vorrichtung zum Verschweissen von thermoplastischen Schläuchen, wobei der Erkennbarkeit einzelner Teile wegen in allen Figuren nicht jeweils alle Teile der Vorrichtungen dargestellt sind. In den Figuren 1 und 3 ist die Vorrichtung ohne Schläuche und in Fig. 2 mit zwei parallel zueinander durchlaufenden Schläuchen 1 und 2 dargestellt.

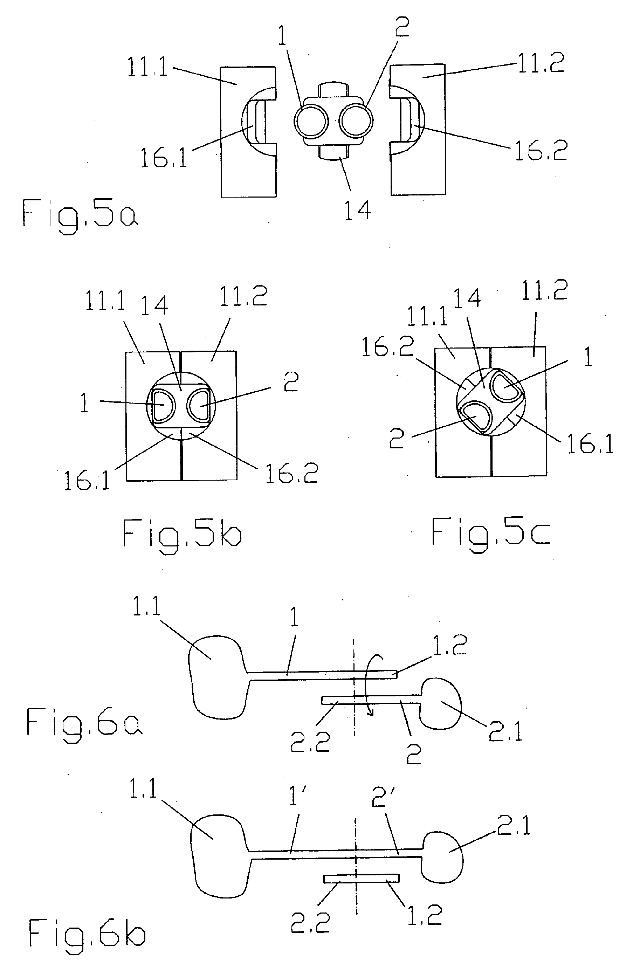

Von den Schläuchen 1 und 2 sind jeweils nur kurze Abschnitte dargestellt. Tatsächlich könnten, wie in Fig. 6a dargestellt, entgegengesetzte Enden der Schläuche 1, 2 z.B. mit Behältern 1.1 bzw. 2.1 und die jeweils anderen Enden 1.2 bzw. 2.2 verschlossen sein. Die Schläuche könnten zudem mit Flüssigkeit gefüllt sein.

Die erfindungsgemässe Vorrichtung weist zwei dicht nebeneinander angeordnete Spanneinheiten 10 und 20 auf, zwischen welche eine beheizbare Klinge 3 zum Durchtrennen und miteinander Verschweissen der Schläuche 1 und 2 eingeschwenkt werden kann.

Beide Spanneinheiten 10 und 20 umfassen jeweils zwei Spannbacken, welche bei der Spanneinheit 10 mit 11.1 und 11.2 und bei der Spanneinheit 20 mit 21.1 und 21.2 bezeichnet sind.

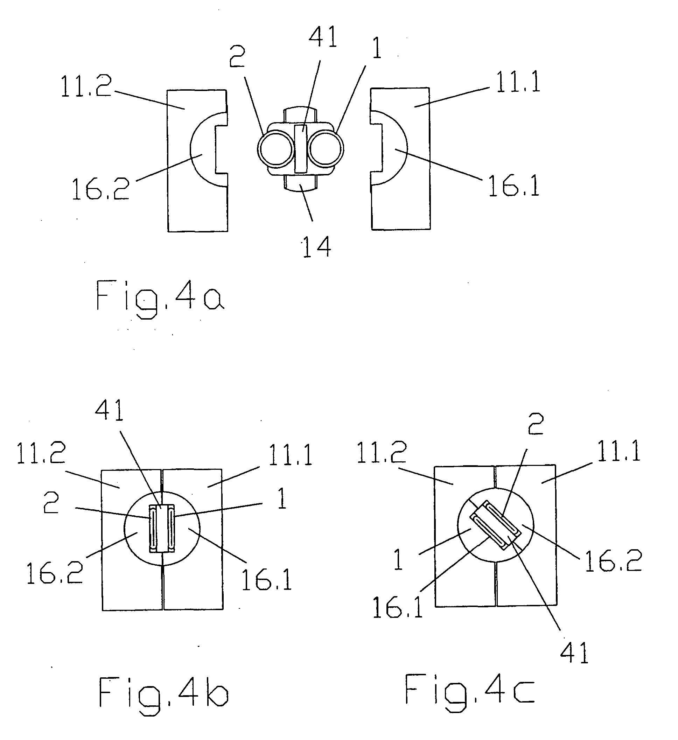

Die Spannbacken 11.1 und 11.2 sind entlang von horizontalen linearen Führungen 12 bzw. 22 gegenüber den an einem Ende dieser Führungen ortsfest montierten Spannbacken 11.1 und 21.1 bewegbar und lassen sich dadurch zwischen ihrer in den Figuren 1 und 2 dargestellten geöffneten Stellung in die Schliesstellung gemäss den Figuren 4b, 4c oder 5b, 5c verstellen. Für diese Verstellung sind zwei in Fig. 3 dargestellte Motoren 13 und 23 vorgesehen, die über Zahnstangen auf die Spannbacken 11.2 und 21.2 einwirken.

Zwischen den Spannbacken ist bei beiden Spanneinheiten 10 und 20 jeweils ein Zwischenstück 14 bzw. 24 angeordnet, wobei diese Zwischenstücke, wie in Fig. 2 ersichtlich, die Schläuche 1 und 2 voneinander trennen. Die Zwischenstücke 14 und 24 sind jeweils auf Halterungen 15 und 25 gehalten, die wie die Spannbacken 11.2 und 21.2 auf den Führungen 11 und 22 linear verschiebbar sind.

In die im folgenden als äussere Spannbacken bezeichneten Spannbacken 11.1 und 11.2 der Spanneinheit 10 sind innere Spannbacken 16.1 und 16.2 eingesetzt. Fig. 4a und Fig. 5a zeigen Ansichten dieser Spanneinheit im geöffneten Zustand. In der Schliessstellung der Spanneinheit 10 gemäss Fig. 4b oder 5b bilden die inneren Spannbacken 16.1 und 16.2 zusammen mit dem von ihnen eingeschlossenen Zwischenstück 14 eine in den äusseren Spannbacken 11.1 und 11.2 drehbare Kapsel. Die Figuren 4c und 5c zeigen die Spannvorrichtung 10 mit dieser Kapsel gegenüber ihrer Darstellung in den Figuren 4b und 5b verdreht.

In der geöffneten Stellung der Spanneinheit 10 sind die beiden inneren Spannbacken 16.1 und 16.2 in den äusseren Spannbacken 11.1 und 11.2 gegen Verdrehen fixiert, wobei dies z.B. durch Stifte erreicht sein kann, welche von den äusseren Spannbacken 11.1 und 11.2 aus in die inneren Spannbacken 16.1 und 16.2 eingreifen und welche Stifte, z.B. gesteuert über eine Kulissenführung, bei Erreichen der Schliessstellung aus den inneren Spannbacken 16.1 und 16.2 zurückgezogen werden. Die Verbindung des Zwischenstücks 14 zu seiner Halterung 15 kann auf entsprechende Weise durch Stifte 31 ausgebildet und bei Erreichen der Schliessstellung aufgehoben werden.

Bezüglich ihrer Kontur gegenüber den Schläuchen 1 und 2 sind die inneren Spannbacken 16.1 und 16.2 der Spanneinheit 10 sowie die Spannbacken 21.1 und 21.2 der Spanneinheit 20 untereinander jeweils identisch ausgebildet. Zusätzlich sind die inneren Spannbacken 16.1 und 16.2 der Spanneinheit 10 rotationssymmetrisch bezüglich halber Umdrehungen. Entsprechendes gilt für die Zwischenstücke 14 und 24, wobei das Zwischenstück 14 auch in zwei um 180° gegeneinander versetzen Drehstellungen mit der Halterung 15 verbindbar ist.

Die inneren Spannbacken 16.1 und 16.2 definieren zusammen mit dem Zwischenstück 14 in der Schliessstellung der Spanneinheit 10 zwei durch das Zwischenstück 14 voneinander getrennte Durchgangsöffnungen für die Schläuche 1 und 2. Entsprechendes gilt für Spanneinheit 20, wobei hier die Spannbacken 21.1 und 21.2 zusammen mit dem Zwischenstück 24 die Durchgangsöffnungen definieren. In den Schliessstellungen beider Spanneinheiten 10 und 20 fluchten ihre Durchgangsöffnungen jeweils miteinander, so dass die Schläuche 1 und 2 durchlaufend in sie eingespannt werden können.

In Längsrichtung der Schläuche weisen die Durchgangsöffnungen jeweils zwei Zonen mit unterschiedlichem Querschnitt auf, nämlich einerseits Quetsch- und andererseits Haltezonen. In den Quetschzonen ist dieser Querschnitt rechteckig und wird auf seinen längeren Seiten begrenzt durch Stege 41 an den Zwischenstücken und durch stegartige Wandungen 42 an den Spannbacken. Die Breite des rechteckigen Querschnitts entlang von dessen kürzeren Seiten entspricht knapp der doppelten Wandstärke der Schläuche 1, 2. Die Dicke der stegartigen Wandungen 42 und damit die Längserstreckung der Quetschzonen beträgt typischerweise nur etwa 1 - 6 mm. An beiden Spanneinheiten 10 und 20 befinden sich die Quetschzonen angrenzend an ihre sich gegenüberliegenden Seiten mit geringstem gegenseitigem Abstand.

In den Haltezonen ist der Querschnitt der Durchgangsöffnungen gegenüber dem rechteckigen Querschnitt in den Quetschzonen jeweils nach aussen hin weg von den Zwischenstücken 14, 24 erweitert, wobei die Zwischenstücke dort ein doppel-c-förmiges, an den Durchmesser der Schläuche angepasstes Profil aufweisen. Die den Zwischenstücken 14, 24 gegenüberliegenden Anlageflächen 43 an den Spannbacken sind demgegenüber eben ausgebildet.

Bei geöffneten Spannbacken können die Schläuche 1 und 2, jeweils von oben in die Spanneinheiten 1, 2 eingelegt und durch Eindrücken in das doppel-c-fömige Profil der Zwischenstücke 14, 24 jeweils bereits auf Höhe von deren Drehachse vorfixiert werden, wie dies aus den Figuren 2 und 4 ersichtlich ist. Beim Verfahren der Spannbacken 11.2 und 21.2 mit dem erwähnten motorischen in die Schliessstellung, wobei die Zwischenstücke 14 und 24 entlang der Führungen 12 bzw. 22 mitbewegt werden, werden die Schläuche, wie in Fig. 4b dargestellt, in den Quetschzonen jeweils bis zu ihrem totalen Verschluss zusammengequetscht. Die längeren Seiten der Durchgangsöffnungen in den Quetschzonen müssen hierzu natürlich ausreichend lang bemessen sein, wobei sie durchaus auch länger als jeweils erforderlich sein können, da die Schläuche durch das doppel-c-förmige Profil der Zwischenstücke 14, 24 in den Haltezonen in den Durchgangsöffnungen zentriert werden. Das Mass dieser Seiten kann daher für unterschiedliche Schlauchdurchmesser in gewissen Grenzen übereinstimmend gewählt werden und muss zur Anpassung der Vorrichtung an unterschiedliche Schlauchdurchmesser nicht variiert werden.

Die erfindungsgemässe Vorrichtung kann insbesondere dazu verwendet werden, die Schläuche 1 und 2 zwischen den beiden Spanneinheiten 10 und 20 zu durchtrennen und ihre dadurch entstehenden Teile über Kreuz miteinander zu verschweissen, ohne dass dabei, wegen des durch das Zusammenquetschen erreichten Verschlusses der Schläuche während des gesamten Vorgangs, in den Schläuchen vorhandene Flüssigkeit in die Umgebung austreten kann.

Damit sich zwischen den Quetschzonen beider Spanneinheiten 10, 20 keine flüssigkeitsgefüllten Taschen ausbilden können, müssen die Spanneinheiten 10, 20 dicht benachbart sein, wobei der gegenseitige Abstand z.B. nur zwischen 3 und 5 mm betragen sollte. Ausserdem müssen dazu die beiden Spanneinheiten 10, 20 nacheinander zugefahren werden.

In der Schliessstellung beider Spanneinheiten werden die Schläuche mit der auf mehrere hundert ° C erhitzten Klinge 3 durchtrennt. Anschliessend wird bei noch eingeschwenkter Klinge die erwähnte Kapsel aus den inneren Spannbacken 16.1, 16.2 und dem Zwischenstück 14 zusammen mit den in ihr eingequetschten Schlauchteilen in der Spanneinheit 10 um 180° gedreht. Ausgeführt werden kann diese Drehung wiederum motorisch, wozu der in den Figuren 1 und 2 mit 50 bezeichnete Motor vorgesehen ist. Dann wird die Klinge 3 ausgeschwenkt und die durch die Drehung neu in Flucht miteinander gebrachten, noch heissen und aufgeschmolzenen Schlauchenden durch geringfügiges Zusammenfahren der beiden Spanneinheiten 10, 20 miteinander verschweisst. Die Führungen und der Antrieb für dieses Zusammenfahren sind in den Figuren nicht dargestellt. Wegen des geringen Weges könnte hierzu eine Excenterscheibe eingesetzt werden.

Ausgehend von einer Anordnung, wie sie in Fig. 6a dargestellt ist, zeigt Fig. 6b das Ergebnis dieses Vorgehens. In Fig. 6b sind die beiden, jeweils um ihre Endstücke gekürzten Schläuche 1' und 2' zu einem durchgehenden, die beiden Behälter 1.1 und 2.1 miteinander verbindenden Schlauch verbunden. Andererseits sind die beiden Endstücke ebenfalls miteinander verbunden und bilden mit ihren verschlossenen Enden 1.2 und 2.2 selbst einen geschlossenen Behälter.

Nach dem wie beschrieben ausgeführten Verschweissen können die Spanneinheiten 10, 20 wieder aufgefahren und die Schläuche 1, 2 aus ihnen entnommen werden. Wegen der erwähnten Rotationsymmetrie der inneren Spannbacken 16.1 und 16.2 und des Zwischenstücks 14 ist dies direkt möglich, ohne dass die genannten Teile zusätzlich, z. B. wieder in ihre Ausgangsstellung, gedreht werden müssen. Die Vorrichtung ist deshalb auch sofort für das Verschweissen weiterer Schläuche einsetzbar.

Die erfindungsgemässe Vorrichtung lässt sich an Schläuche mit unterschiedlichem Durchmesser und/oder Wandstärke lediglich durch Auswechseln der Zwischenstücke 14, 24 anpassen. Solche zum Verschweissen geeignete Schläuche weisen typischerweise einen Durchmesser zwischen 1/8 und 3 Zoll auf. Wie bereits erwähnt, kann dass Mass der längeren Seiten der Durchgangsöffnungen in den Quetschzonen einheitlich für verschiedene Schlauchdurchmesser gewählt werden. In den Haltezonen genügt die Anpassung des doppel-c-förmigen Profils der Zwischenstücke 14, 24 an den jeweiligen Schlauchdurchmesser, da die korrespondierenden flachen Anlageflächen 43 an den Spannbacken für alle Schlauchdurchmesser geeignet sind. Zur Anpassung an Schläuche mit unterschiedlichen Wandstärken genügt es, die Dicke der Zwischenstücke 14, 24 zu verändern.

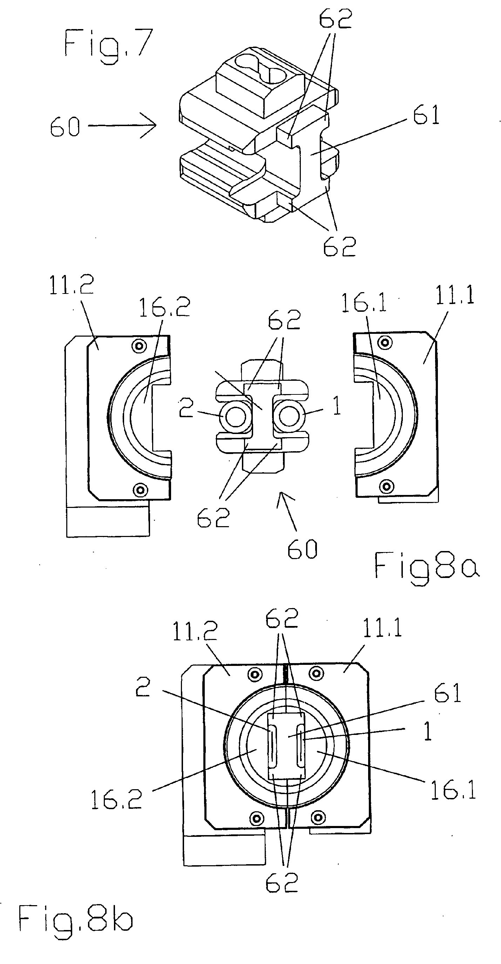

Die Fig. 7 zeigt eine Variante des Zwischenstücks mit doppel-T-förmigem Steg. Fig. 8 zeigt die Spannbacken 11.1, 11.2, 16.1, 16.2 der Spanneinheit 10 mit dem Zwischenstück aus Fig. 7 und mit zwei eingelegten Schläuchen 1, 2, wobei Fig. 8a die geöffnete Stellung und Fig. 8b die Schliessstellung darstellt.

Das Zwischenstück 60 weist einen Steg 61 auf, welcher in der Schliessstellung der Vorrichtung mittig zwischen den Durchgangsöffnungen im Bereich der Quetschzonen angeordnet ist und jeweils eine der längeren Rechteckseiten der rechteckigen Querschnitte darstellt. Bei unveränderter Dimensionierung der Spannbacken ist die Breite der Durchgangsöffnungen im Bereich der Quetschzonen durch die Breite des Stegs 61 definiert. Dies gilt auch für das Zwischenstück aus Fig. 4.

Im Unterschied zum Zwischenstück 14 aus Fig. 4 weist der Steg 61 des Zwischenstücks 60 zusätzlich beidseitig jeweils zwei Vorsprünge 62 auf, so dass sich ein doppel-T-förmiges Profil ergibt. Diese Vorsprünge 62 stellen die kürzeren Rechteckseiten der rechteckigen Querschnitte der Durchgangsöffnungen im Bereich der Quetschzonen dar und definieren die Länge der rechteckigen Querschnitte der Durchgangsöffnungen im Bereich der Quetschzonen.

Je nach Dimensionierung des Stegs 61 und der Vorsprünge 62 und somit des rechteckigen Querschnitts der Durchgangsöffnungen im Bereich der Quetschzone kann die Vorrichtung durch schlichtes Austauschen der Zwischenstücke an Schläuche 1, 2 mit verschiedenen Durchmessern und Wandstärken angepasst werden. Die Dimensionen der verschiedenen Spannbacken müssen dafür nicht geändert werden.

Dabei wird die Breite des Stegs 61 vorzugsweise so gewählt, dass die Breite des rechteckigen Querschnitts der Durchgangsöffnung im Bereich der Quetschzonen 50 - 70 % der doppelten Wandstärke der zu quetschenden Schläuche 1, 2 beträgt. Dies kann auch bei den vorangehend beschriebenen Zwischenstücken 14 so gemacht werden. Die Vorsprünge 62 werden vorzugsweise so gewählt, dass die Länge des rechteckigen Querschnitts der Durchgangsöffnung im Bereich der Quetschzonen 120 - 140 % des Durchmessers der zu quetschenden Schläuche 1, 2 beträgt.

### BEZEICHNUNGSLISTE

- 1: Schlauch
- 2: Schlauch
- 1.1: Behälter
- 2.1: Behälter
- 1.2: verschlossenes Ende
- 2.2: verschlossenes Ende
- 3: beheizbare Klinge
- 10: Spanneinheit
- 20: Spanneinheit
- 11.1: (äussere) Spannbacken
- 11.2: (äussere) Spannbacken
- 21.1: Spannbacken
- 21.2: Spannbacken
- 12: lineare Führungen
- 22: lineare Führungen
- 13: Motor
- 23: Motor
- 14: Zwischenstück
- 24: Zwischenstück
- 15: Halterung
- 25: Halterung
- 16.1: innere Spannbacken
- 16.2: innere Spannbacken
- 31: Stifte
- 41: Stege an den Zwischenstücken
- 42: stegartige Wandungen
- 43: Anlageflächen
- 50: Motor
- 60: Zwischenstück
- 61: Steg am Zwischenstück 60
- 62: Vorsprünge

## Patentansprüche

1. Vorrichtung zum Verschweissen von thermoplastischen Schläuchen (1, 2) mit zwei gegeneinander in Teilen verdrehbaren und verschiebbaren, jeweils zwei Spannbacken (11.1, 11.2, 16.1, 16.2; 21.1, 21.2) aufweisenden Spanneinheiten (10, 20) mit Durchgangsöffnungen zwischen den Spannbacken, in welche die Schläuche parallel zueinander durchlaufend eingelegt und quer zueinander gequetscht werden können, und mit einer beheizbaren Klinge (3) zum Durchschneiden der Schläuche und Aufschmelzen ihrer Schnittkanten zwischen den Spanneinheiten, **dadurch gekennzeichnet, dass** zwischen den Spannbacken der Spanneinheiten, eingespannte Schläuche voneinander trennend, jeweils ein Zwischenstück (14, 24) angeordnet ist und dass mindestens eine (10) der beiden Spanneinheiten innere sowie äussere Spannbacken aufweist, wobei die inneren Spannbacken (16.1, 16.2) von den äusseren Spannbacken (11.1, 11.2) gehalten, gemeinsam mit diesen verstellbar und in einer Schliessstellung miteinander sowie zusammen mit dem von ihnen eingeschlossenen Zwischenstück (14) in den äusseren Spannbacken drehbar sind.

2. Vorrichtung nach Anspruch 1, **dadurch gekennzeichnet, dass** die Durchgangsöffnungen im Bereich von Quetschzonen einen rechteckigen Querschnitt aufweisen, wobei die längeren Rechteckseiten dieser Querschnitte parallel zueinander ausgerichtet sind.

3. Vorrichtung nach Anspruch 2, **dadurch gekennzeichnet, dass** das Zwischenstück (14, 24; 60) einen Steg (41; 61) aufweist, wobei in der Schliessstellung der Steg (41; 61) mittig zwischen den Durchgangsöffnungen im Bereich der Quetschzonen angeordnet ist und jeweils eine der längeren Rechteckseiten der rechteckigen Querschnitte darstellt.

4. Vorrichtung nach Anspruch 3, **dadurch gekennzeichnet, dass** der Steg (61) beidseitig jeweils zwei Vorsprünge (62) aufweist, so dass sich ein doppel T-förmiges Profil ergibt, wobei die Vorsprünge (62) die kürzeren Rechteckseiten der rechteckigen Querschnitte der Durchgangsöffnungen im Bereich der Quetschzonen bilden und die Länge der rechteckigen Querschnitte der Durchgangsöffnungen im Bereich der Quetschzonen festlegen.

5. Vorrichtung nach einem der Ansprüche 2 - 4, **dadurch gekennzeichnet, dass** die Breite des rechteckigen Querschnitts der Durchgangsöffnung im Bereich der Quetschzonen 50 - 70 % der doppelten Wandstärke der zu quetschenden Schläuche beträgt.

6. Vorrichtung nach einem der Ansprüche 2 - 5, **dadurch gekennzeichnet, dass** die Länge des rechteckigen Querschnitts der Durchgangsöffnung im Bereich der Quetschzonen 120 - 140 % des Durchmessers der zu quetschenden Schläuche beträgt.

7. Vorrichtung nach Anspruch 2, **dadurch gekennzeichnet, dass** die Durchgangsöffnungen im Bereich von Haltezonen einen gegenüber dem Querschnitt in den Quetschzonen erweiterten Querschnitt aufweisen, wobei dieser Querschnitt jeweils in Richtung weg von den Zwischenstücken (14, 24; 60) erweitert ist.

8. Vorrichtung nach Anspruch 7, **dadurch gekennzeichnet, dass** die Zwischenstücke (14, 24; 60) im Bereich der Haltezonen jeweils ein doppel c-förmiges Profil zur Aufnahme und Vorfixierung der Schläuche aufweisen.

9. Vorrichtung nach Anspruch 7 oder 8, **dadurch gekennzeichnet, dass** die Spannbacken (21.1, 21.2) und/oder die inneren Spannbacken (16.1, 16.2) im Bereich der Haltezonen jeweils eine flache Anlagefläche (43) gegenüber den Schläuchen aufweisen.

10. Vorrichtung nach einem der Ansprüche 1-9, **dadurch gekennzeichnet, dass** die inneren Spannbacken (16.2, 16.2) in den äusseren Spannbacken (11.1, 11.2) ausserhalb der Schliessstellung gegen Verdrehen fixiert sind.

11. Vorrichtung nach einem der Ansprüche 1-10, **dadurch gekennzeichnet, dass** die Zwischenstücke (14, 24) jeweils auf einer Halterung (15, 25) gehalten sind, wobei die Verbindung des Zwischenstücks (14) zwischen den inneren (16.1, 16.2) zu seiner Halterung (15) in der Schliessstellung gelöst ist.

12. Vorrichtung nach einem der Ansprüche 1-11, **dadurch gekennzeichnet, dass** jeweils einer der Spannbacken (21.1) bzw. der äusseren Spannbacken (11.1) der Spanneinheiten (10, 20) ortsfest montiert und zum Einlegen in die bzw. Entnehmen der Schläuche aus den Durchgangsöffnungen jeweils nur der andere Spannbacken (11.2, 21.2) sowie die Zwischenstücke (14, 24; 60) an vorzugsweise linearen Führungen (12, 22) vorzugsweise motorisch bewegbar montiert ist.

13. Vorrichtung nach einem der Ansprüche 1-12, **dadurch gekennzeichnet, dass** die Spanneinheit (10) mit den inneren (16.1, 16.2) und den äusseren Spannbacken (11.1, 11.2) einschliesslich des in ihr angeordneten Zwischenstücks (14) rotationssymmetrisch bezüglich halber Umdrehungen der inneren Spannbacken (16.1, 16.2) und des Zwischenstücks (14) in den äusseren Spannbacken (11.1, 11.2) ist.

14. Vorrichtung nach einem der Ansprüche 1-13, **dadurch gekennzeichnet, dass** der Abstand der Quetschzonen beider Spanneinheiten (10) beim Durchschneiden der Schläuche 3-6 mm, vorzugsweise von 5 mm, beträgt und dass dieser Abstand zum Verschweissen der Schläuche um einige mm verringerbar ist.

## Claims

1. Device for welding thermoplastic tubes (1, 2), having two clamping units (10, 20), which are rotatable and displaceable in part with respect to one another and each have two clamping jaws (11.1, 11.2, 16.1, 16.2; 21.1, 21.2), having through-passage openings between the clamping jaws, into which the tubes are inserted in a manner passing through parallel to one another and can be squeezed transversely to one another, and having a heatable blade (3) for cutting through the tubes and fusing their cut edges between the clamping units, **characterized in that** in each case one intermediate piece (14, 24) is arranged between the clamping jaws of the clamping units, separating clamped-in tubes from one another, and **in that** at least one (10) of the two clamping units has inner and outer clamping jaws, wherein the inner clamping jaws (16.1, 16.2) are held by the outer clamping jaws (11.1, 11.2), are adjustable together with the latter and in a closed position are rotatable with one another and together with the intermediate piece (14) enclosed by them, in the outer clamping jaws.

2. Device according to Claim 1, **characterized in that** the through-passage openings have a rectangular cross section in the region of a squeezing zone, wherein the longer rectangle sides of this cross section are oriented parallel to one another.

3. Device according to Claim 2, **characterized in that** the intermediate piece (14, 24; 60) has a rib (41; 61), wherein, in the closed position, the rib (41; 61) is arranged centrally between the through-passage openings in the region of the squeezing zones and represents in each case one of the longer rectangle sides of the rectangular cross sections.

4. Device according to Claim 3, **characterized in that** the rib (61) has in each case two protrusions (62) on both sides, so that a double T-shaped profile results, wherein the protrusions (62) form the shorter rectangle sides of the rectangular cross sections of the through-passage openings in the region of the squeezing zones and define the length of the rectangular cross sections of the through-passage openings in the region of the squeezing zones.

5. Device according to one of Claims 2-4, **characterized in that** the width of the rectangular cross section of the through-passage opening in the region of the squeezing zones is 50-70% of twice the wall thickness of the tubes to be squeezed.

6. Device according to one of Claims 2-5, **characterized in that** the length of the rectangular cross section of the through-passage opening in the region of the squeezing zones is 120-140% of the diameter of the tubes to be squeezed.

7. Device according to Claim 2, **characterized in that** the through-passage openings have in the region of holding zones a cross section that is expanded compared with the cross section in the squeezing zones, wherein this cross section is expanded in each case in the direction away from the intermediate pieces (14, 24; 60).

8. Device according to Claim 7, **characterized in that** the intermediate pieces (14, 24; 60) each have in the region of the holding zones a double C-shaped profile for receiving and prefixing the tubes.

9. Device according to Claim 7 or 8, **characterized in that** the clamping jaws (21.1, 21.2) and/or the inner clamping jaws (16.1, 16.2) each have in the region of the holding zones a flat abutment surface (43) with respect to the tubes.

10. Device according to one of Claims 1-9, **characterized in that** the inner clamping jaws (16.1, 16.2) are fixed in the outer clamping jaws (11.1, 11.2) so as to be prevented from rotating outside the closed position.

11. Device according to one of Claims 1-10, **characterized in that** the intermediate pieces (14, 24) are each held on a holder (15, 25), wherein the connection of the intermediate piece (14) between the inner clamping jaws (16.1, 16.2) to its holder (15) is released in the closed position.

12. Device according to one of Claims 1-11, **characterized in that** in each case one of the clamping jaws (21.1) or of the outer clamping jaws (11.1) of the clamping units (10, 20) is mounted in a fixed position and in order to insert the tubes into or remove them from the through-passage openings, in each case only the other clamping jaw (11.2, 2 1.2) and the intermediate pieces (14, 24; 60) are mounted movably, preferably by way of a motor, on preferably linear guides (12, 22).

13. Device according to one of Claims 1-12, **characterized in that** the clamping unit (10) having the inner clamping jaws (16.1, 16.2) and the outer clamping jaws (11.1, 11.2), including the intermediate piece (14) arranged in it, is rotationally symmetrical with regard to half revolutions of the inner clamping jaws (16.1, 16.2) and of the intermediate piece (14) in the outer clamping jaws (11.1,11.2).

14. Device according to one of Claims 1-13, **characterized in that** the distance between the squeezing zones of the two clamping units (10) when the tubes are cut through is 3-6 mm, preferably 5 mm, and **in that** this distance is reducible by a few mm in order to weld the tubes.

## Revendications

1. Dispositif de soudage de tubes thermoplastiques (1,2) comprenant deux unités de serrage (10, 20) pouvant être tournées et coulissées l'une par rapport à l'autre en partie, présentant à chaque fois deux mâchoires de serrage (11.1, 11.2, 16.1, 16.2 ; 21.1, 21.2), avec des ouvertures de passage entre les mâchoires de serrage, dans lesquelles les tubes s'étendant parallèlement les uns aux autres peuvent être introduits et écrasés transversalement les uns aux autres, et comprenant une lame chauffable (3) pour couper les tubes et faire fondre leurs arêtes de coupe entre les unités de serrage, **caractérisé en ce qu'**entre les mâchoires de serrage des unités de serrage, est disposée à chaque fois une pièce intermédiaire (14, 24) séparant les uns des autres les tubes serrés, et **en ce qu'**au moins l'une (10) des deux unités de serrage présente des mâchoires de serrage intérieures et extérieures, les mâchoires de serrage intérieures (16.1, 16.2), maintenues par les mâchoires de serrage extérieures (11.1, 11.2), pouvant être réglées conjointement avec celles-ci et pouvant être tournées dans les mâchoires de serrage extérieures dans une position de fermeture l'une avec l'autre ainsi que conjointement avec la pièce intermédiaire (14) qu'elles enserrent.

2. Dispositif selon la revendication 1, **caractérisé en ce que** les ouvertures de passage présentent dans la région de zones d'écrasement une section transversale rectangulaire, les côtés rectangulaires plus longs de ces sections transversales étant orientés parallèlement les uns aux autres.

3. Dispositif selon la revendication 2, **caractérisé en ce que** la pièce intermédiaire (14,24; 60) présente une nervure (41 ; 61), la nervure (41; 61) étant disposée dans la position de fermeture centralement entre les ouvertures de passage dans la région des zones d'écrasement et constituant à chaque fois l'un des côtés longs du rectangle des sections transversales rectangulaires.

4. Dispositif selon la revendication 3, **caractérisé en ce que** la nervure (61) présente de chaque côté à chaque fois deux saillies (62), de telle sorte qu'il en résulte un profil en forme de double T, les saillies (62) formant les côtés courts du rectangle des sections transversales rectangulaires des ouvertures de passage dans la région des zones d'écrasement et fixant la longueur des sections transversales rectangulaires des ouvertures de passage dans la région des zones d'écrasement.

5. Dispositif selon l'une quelconque des revendications 2 à 4, **caractérisé en ce que** la largeur de la section transversale rectangulaire de l'ouverture de passage dans la région des zones d'écrasement vaut 50 à 70 % de la double épaisseur de paroi des tubes à écraser.

6. Dispositif selon l'une quelconque des revendications 2 à 5, **caractérisé en ce que** la longueur de la section transversale rectangulaire de l'ouverture de passage dans la région des zones d'écrasement vaut 120 à 140 % du diamètre des tubes à écraser.

7. Dispositif selon la revendication 2, **caractérisé en ce que** les ouvertures de passage dans la région de zones de retenue présentent une section transversale élargie par rapport à la section transversale dans les zones d'écrasement, cette section transversale étant à chaque fois élargie dans la région s'éloignant des pièces intermédiaire (14, 24 ; 60).

8. Dispositif selon la revendication 7, **caractérisé en ce que** les pièces intermédiaires (14, 24 ; 60) dans la région des zones de retenue présentent à chaque fois un profil en forme de double c pour recevoir et fixer préalablement les tubes.

9. Dispositif selon la revendication 7 ou 8, **caractérisé en ce que** les mâchoires de serrage (21.1, 21.2) et/ou les mâchoires de serrage intérieures (16.1, 16.2) dans la région des zones de retenue présentent à chaque fois une surface d'appui (43) par rapport aux tubes.

10. Dispositif selon l'une quelconque des revendications 1 à 9, **caractérisé en ce que** les mâchoires de serrage intérieures (16.1, 16.2) sont fixées contre toute rotation dans les mâchoires de serrage extérieures (11.1, 11.2) à l'extérieur de la position de fermeture.

11. Dispositif selon l'une quelconque des revendications 1 à 10, **caractérisé en ce que** les pièces intermédiaires (14, 24) sont maintenues à chaque fois sur une fixation (15, 25), la connexion de la pièce intermédiaire (14) entre les mâchoires de serrage intérieures (16.1, 16.2) et sa fixation (15) étant desserrée dans la position de fermeture.

12. Dispositif selon l'une quelconque des revendications 1 à 11, **caractérisé en ce que** l'une respective des mâchoires de serrage (21.1) ou des mâchoires de serrage extérieures (11.1) des unités de serrage (10, 20) est montée fixement et pour l'introduction ou le retrait des tubes dans les ouvertures de passage ou hors de celles-ci, à chaque fois seulement l'autre mâchoire de serrage (11.2, 21.2) ainsi que les pièces intermédiaires (14, 24 ; 60) sont montées sur des guides de préférence linéaires (12, 22) de manière déplaçable de préférence par un moteur.

13. Dispositif selon l'une quelconque des revendications 1 à 12, **caractérisé en ce que** l'unité de serrage (10) avec les mâchoires de serrage intérieures (16.1, 16.2) et les mâchoires de serrage extérieures (11.1, 11.2), y compris la pièce intermédiaire (14) disposée dans celle-ci, est réalisée avec une symétrie de révolution par rapport à des demi-rotations des mâchoires de serrage intérieures (16.1, 16.2) et de la pièce intermédiaire (14) dans les mâchoires de serrage intérieures (11.1,11.2).

14. Dispositif selon l'une quelconque des revendications 1 à 13, **caractérisé en ce que** la distance des zones d'écrasement des deux unités de serrage **(10)** lors de la découpe des tubes est de 3 à 6 mm, de préférence de 5 mm, et **en ce que** cette distance peut être réduite de quelques mm pour le soudage des tubes.
